# EUROPEAN PATENT SPECIFICATION

(11) **EP 0 556 908 B1**
(45) Date of publication and mention of the grant of the patent: **09.10.1996**
(21) Application number: 93200393.2
(22) Date of filing: 12.02.1993
(51) Int. Cl.: A61F 6/14, A61F 2/00

(54) **Intrauterine and/or intravaginal device**
Intrauterine und/oder intravaginale Vorrichtung
Dispositif intrauterin et/ou intravaginal

(30) Priority: 19.02.1992 NL 9200308
(43) Date of publication of application: 25.08.1993
(73) Proprietor: TECHNOLOGY DEVELOPMENT GROUP INC., Tortola (VG)
(72) Inventor: Van Os, Willem Arthur Adriaan, Monte Carlo, Monaco 98000 (MC)
(74) Representative: Kupecz, Arpad

(56) References cited:
- DE-A- 3 518 402
- DE-A- 3 720 858
- FR-A- 2 165 714
- US-A- 4 102 998
- US-A- 4 246 896
- US-A- 4 331 131

## Description

The present invention relates to an intrauterine and/or intravaginal device with an elongated stem.

FR-A-7144512 discloses an intrauterine contraceptive device comprising an elongated stem.

Such an intrauterine device is known from the Dutch patent 173707, which device is a contraceptive. The known device comprises an elongated stem, whereby one of the extremities has two elastic, self-supported arms protruding sideways from either side of the stem.

This intrauterine contraceptive is kept in position in the uterus partly with the aid of small protrusions attached to the arms. However, this device has the disadvantage that the small protrusions lodge themselves in the endometrium, with the result that on removal of the device from the uterus, bleeding may occur where the protrusions were lodged. Such bleeding may cause infections, which is obviously undesirable.

Further, the European patent specification 0100924 describes a intrauterine pessary, comprising an elongated stem, which at the extremities is provided with an anchoring device in the form of a spiral. The extremity of the anchoring spiral is, as it were, introduced into the fundus of the uterine muscle. It will be obvious that insertion of such a device must be carried out with great care in order to prevent the spiral being pushed too far into the fundus, which could cause serious injuries. Injury to the uterine fundus can also occur in the case of removal of such a device from the uterus, which is not only painful but can also cause infections of the uterus as well as the cervix of the woman.

The aim of the invention is to provide a new intrauterine and/or intravaginal device whereby the above-mentioned disadvantages are effectively eliminated.

To this end the invention comprises an intrauterine and/or intravaginal device comprising an elongated stem which is provided with anchoring means, **characterized** in that said anchoring means forms an integral part of the stem and is constructed for piercing the cervix.

Surprisingly it has been shown that anchoring such a device in the cervix instead of the uterus offers considerable advantages compared with the known method for anchoring.

The device is anchored at a distance of about 1.5 cm from the cervical opening, in sight, in other words the person inserting it is able to see what he is doing, which is a fact that may be mentioned as an advantage of anchoring in the cervix. As the cervix is easier to see it is easier to disinfect, consequently fewer infections are to be expected after the anchoring means is positioned. Obviously this is not the case with the known devices described above, whereby one has to work by feel, suffering all the possible harmful consequences, such as piercing the uterine fundus.

The device of the invention may advantageously be applied as an intrauterine as well as an intravaginal device, in which case the elongated stem is anchored at about its middle so that about half of the stem protrudes into the uterus respectively into the uterine canal, while the other half protrudes into the vagina.

The device according to the invention can, however, be either an intrauterine device, or an intravaginal device, in which latter case one end of the stem is anchored in the cervix.

As anchoring means for the elongated stem of the device one can use a knot, screw, thread, etc.

Usually the anchoring means is an integral part of the stem of the device and is preferably made from a metal or metal alloy that is acceptable for the body.

In practice good results are obtained when the anchoring means and the stem are made from stainless steel.

However, it is also possible to make the anchoring means and the stem from plastic that is acceptable for the body, for instance from poly-tetra-fluor-ethylene (PTFE) sold under the tradename Teflon.

Insertion of such a device according to the invention can be carried out in sight, with the aid of a somewhat modified tenaculum of a known type with which the cervix is hooked up. To this purpose the forceps are modified such that they can be used not only for insertion but also for pricking the anchoring means into the cervical wall, which pricking is an essential feature of the invention.

It has been shown that such a pricking operation is almost painless, as the cervical wall is relatively not very sensitive.

It will be obvious that the device of the invention can serve for various purposes.

In the first place the device of the invention is especially suitable as intrauterine device applied as contraceptive.

To this purpose the elongated stem is provided with a means to prevent fertilization in the shape of a spiral copper wire or wire provided with a layer of copper, copper rings or cases. However, the stem can also be loaded with a contraceptive hormone preparation.

The intrauterine device of the invention can also serve as uterine device for radiotherapy in the treatment of carcinoma.

The intrauterine device of the invention can also be loaded with drugs against infections in the uterus.

In the case that the device of the invention is an intravaginal device it can serve as contraceptive. For that purpose the elongated stem is loaded with substances effective as contraceptives, such as copper or hormones or other drugs.

The intravaginal device can also be provided with a sponge disc at its extremity, loaded with a spermicide together with or without an antibiotic, antimycotic, etc.

A special embodiment of the intravaginal device of the invention is an incontinence device, whereby the elongated stem is provided with a ring having a diameter such that the urethra is pushed upward, with the result that interim urine discharge is limited to a minimum. An important advantage of the device of the invention is that the ring is fixed, so that it moves less easily with the result that incontinence, which is such a nuisance to a woman, is permanently counteracted.

It goes without saying that if the device of the invention is at the same time an intrauterine and an intravaginal device, such a device can be put to a great number of uses such as contraceptive, i.e. as contraceptive in the uterus, while in the vagina the device serves as a medium for treating patients against infections, or the other way round, the device serves for the prevention of pregnancy. Moreover, with such a device it is possible to treat carcinoma in the uterus as well as in the vagina.

The invention is elucidated with the aid of the following drawings:
Fig. 1 shows a diagram of the uterus and vagina with the intrauterine and intravaginal device of the invention anchored therein.
Fig. 2 shows an intrauterine device according to the invention.
Fig. 3 shows an intravaginal device according to the invention.
Fig. 4 shows an intravaginal device according to the invention, whereby the elongated stem is provided with a sponge disc.
Fig. 5 shows an intravaginal device according to the invention, which is suitable for the prevention of incontinence in the woman.
Fig. 6 also shows an intravaginal device from the side, which is suitable as device for the treatment of incontinence in the woman, while
Fig. 6a is a view from above of the device as in Fig. 6.

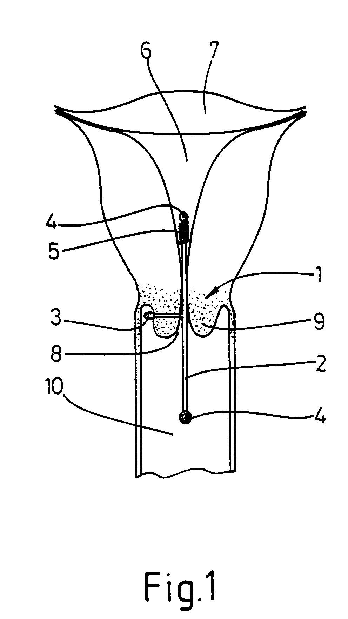

In Fig. 1 the device according to the invention is indicated by reference number 1, which Figure shows how the device 1 is anchored in the cervix 9 with the aid of the anchoring means 3. This embodiment of the device 1 of the invention is an intrauterine as well as an intravaginal device, which can be clearly seen in Fig. 1. The elongated stem 2 is anchored in the cervix 9 at about its middle, with the aid of anchoring means 3, which anchoring means has a pointed end so that during insertion of the device according to the invention the anchoring means can easily be pricked through the cervix without much pain for the woman. Moreover, the end of the anchoring means is shaped such that after the anchoring means has been pricked through the cervix the anchoring means will remain in place. To this end the extremity of the anchoring means is executed such that after the anchoring means has been inserted it is anchored in the cervix.

In this embodiment the end of the anchoring means has a slight thickening in order to ensure that the anchoring means remains in place in the cervix.

One half of the elongated stem 2 is located in the uterus 6, of which the top side is called the fundus uteri 7. The elongated stem 2 protrudes via the cervical canal 8 into the uterus 6. In this embodiment the part of the elongated stem 2 protruding into the uterus is provided with a copper wire 5 in the shape of a spiral, while the end of the elongated stem 2 is provided with a thickening 4 to prevent the copper wire spiral from sliding off the stem and to prevent injury of the uterus. In this embodiment the device according to the invention is suitable as contraceptive. In addition the stem may be loaded with drugs against infection in the uterus or with chemical contraceptives. The part of the elongated stem protruding into the vagina 10 also has a thickening 4, in order to prevent injury to the vagina. The elongated stem in the vagina 10 may be loaded with drugs to combat infection in the vagina or to combat fungus. It should be noted that the device can also serve as device for radiotherapy in the treatment of carcinoma in the uterus as well as in the vagina.

In this embodiment the anchoring means forms an integral part of the stem and is made from stainless steel. Instead of stainless steel one can also use another metal and/or metal alloy that is accepted by the body. Depending on the application of the device it may also be made from plastic that is accepted by the body, as for instance Teflon.

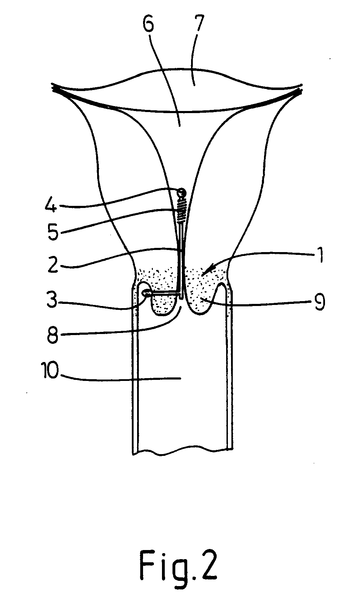

Fig. 2 shows an intrauterine device according to the invention, in which the elongated stem 2 is located in the uterus 6 while the device is anchored in the cervix 9 with the aid of the anchoring means 3. This intrauterine device is provided with a copper wire spiral 5 and thus serves as contraceptive. In addition the device can be loaded with a spermicide together with or without an antibiotic, an antimycotic, etc. The device can also be rendered suitable for radiation treatment of the uterus in cancer therapy.

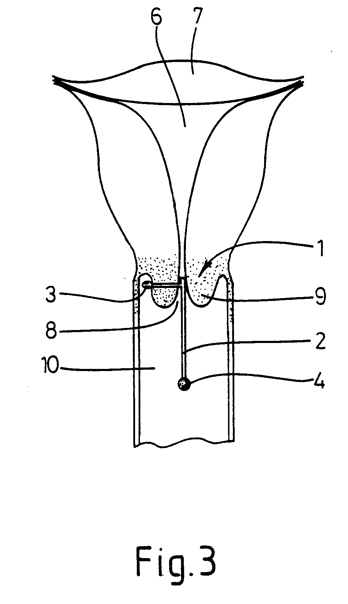

Fig. 3 to 6 describe intravaginal devices according to the invention, whereby in Fig. 3 the elongated stem 2 is located in the vagina 10. The device can serve among other things as a contraceptive or as device for radiotherapy in the treatment of carcinoma of the vagina 10.

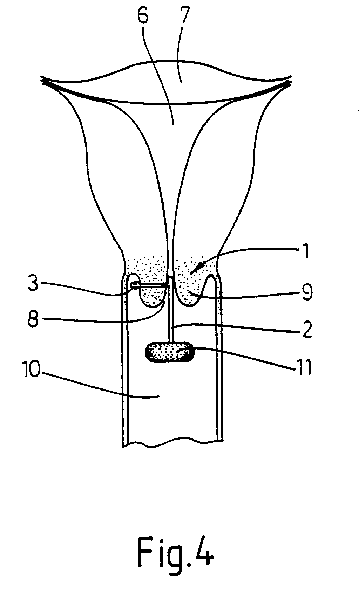

A special embodiment of the device according to the invention is illustrated in Fig. 4, whereby the end of the elongated stem 2 is provided with a sponge disc 11, which is loaded with a spermicide together with or without an antibiotic, an antimycotic, etc. Such a device is especially suitable for the prevention of pregnancy and/or the treatment of infections in the vagina.

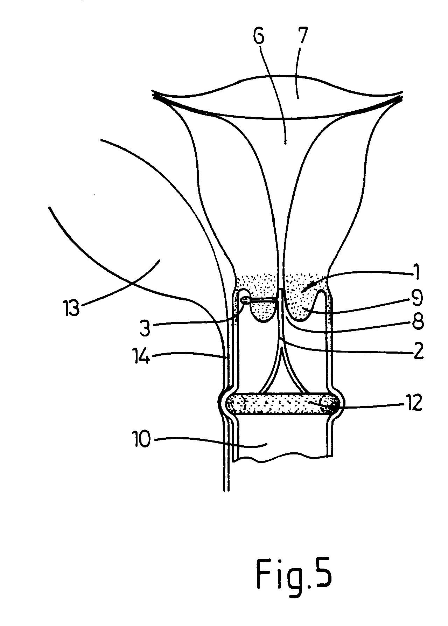

The device according to Fig. 5 is a so-called anti-incontinence device, for which purpose the elongated stem 2 is provided with a ring 12 with a diameter such that the urethra 14 of the urine bladder 13 is pushed upward, limiting spontaneous interim urine discharge to a minimum. The ring, which in this embodiment is made from a plastic with shape memory, will after insertion into and under the influence of the prevailing temperature and humidity in the vagina 10 be caused to expand and will thus exercise its favourable effect on the urethra.

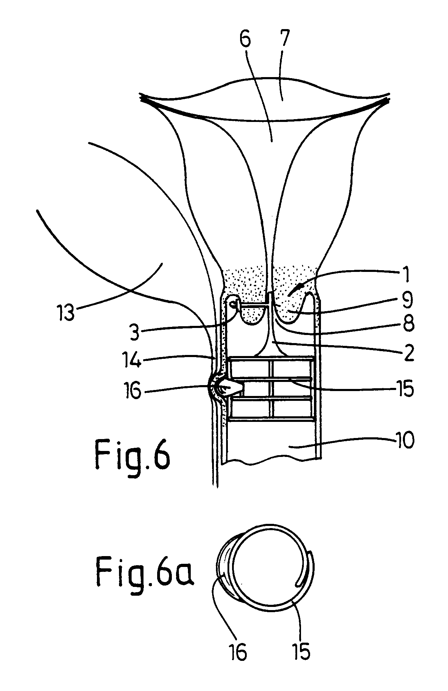

The device according to Fig. 6 is also a device for the treatment of incontinence, whereby the elongated stem 2 comprises a basket 15 made from a plastic with shape memory of the kind discussed in relation to Fig. 5.

In this embodiment Fig. 6a shows clearly that the basket is provided with a thickening 16, which under the influence of the temperature and humidity prevailing in the vagina 10 by expansion of the basket pushes the urethra upward, thereby limiting interim urine discharge to a minimum.

In this embodiment of the invention the anchoring means 3 is at its end provided with a nipple, ensuring permanent fixation in the cervix 9.

Attention is drawn to the fact that the device of the invention can easily be inserted with the aid of a modified tenaculum of the known type, with which the cervix is hooked up.

Finally, it should be noted that the device of the invention is not limited to the embodiments as shown in Figs. 1 to 6.

## Claims

1. An intrauterine and/or intravaginal device comprising an elongated stem, which is provided with anchoring means, **characterized** in that said anchoring means forms an integral part of the stem and is constructed for piercing the cervix.

2. An intrauterine device according to claim 1, **characterized** in that the elongated stem is constructed for being anchored in the cervix at about its middle such that in use about half of the stem protrudes into the uterus respectively the uterine canal, while the other half is in the vagina.

3. An intrauterine or intravaginal device according to claim 1, **characterized** in that one end of the stem is constructed for being anchored in the cervix.

4. A device according to claims 1-3, **characterized** in that the anchoring means is a knot, screw, thread, etc.

5. A device according to claim 1, **characterized** in that the anchoring device and the stem are made from metal and/or metal alloy which is acceptable for the body.

6. A device according to claim 1, **characterized** in that the anchoring means and stem are made from stainless steel.

7. A device according to claims 1-4, **characterized** in that the anchoring means and stem are made from plastic which is acceptable for the body.

8. A device according to claims 1-4 and 7, **characterized** in that the anchoring means and stem are made from PTFE.

9. An intrauterine device according to claims 1 and 3-8, **characterized** in that it is a contraceptive.

10. A device according to claim 9, **characterized** in that the elongated stem of the device is provided with a means to prevent fertilization in the form of a spiral of copper wire or layer of copper, or copper rings or cases.

11. A device according to claims 1 and 3-8, **characterized** in that it is a device for radiation treatment of the uterus.

12. A device according to claims 1 and 3-8, **characterized** in that it is a device which is loaded with drugs against infections in the uterus.

13. An intravaginal device according to claims 1 and 3-8, **characterized** in that the device is a contraceptive.

14. A device according to claim 13, **characterized** in that the elongated stem is loaded with copper or hormones or other drugs.

15. A device according to claim 13, **characterized** in that the stem is provided with a sponge disc loaded with a spermicide together with or without an antibiotic, an anti-mycotic drug, etc.

16. A device according to claims 1 and 3-8, **characterized** in that it is a device for the treatment of incontinence and to that end is provided with a ring with a diameter such that the urethra is pushed upward.

## Patentansprüche

1. Intrauterine und/oder intravaginale Vorrichtung mit einem länglichen Stab, der mit einer Verankerungseinrichtung versehen ist, dadurch gekennzeichet, daß die Verankerungseinrichtung einen integralen Teil des Stabs bildet und zum Durchstechen der Zervix konstruiert ist.

2. Intrauterine Vorrichtung nach Anspruch 1, dadurch gekennzeichnet, daß der längliche Stab zur Verankerung in der Zervix an etwa seiner Mitte derart konstruiert ist, daß etwa die Hälfte des Stabs in den Uterus bzw. den Uteruskanal vorsteht, während die andere Hälfte sich in der Vagina befindet.

3. Intrauterine oder intravaginale Vorrichtung nach Anspruch 1, dadurch gekennzeichnet, daß ein Ende des Stabs zur Verankerung in der Zervix konstruiert ist.

4. Vorrichtung nach den Ansprüchen 1 bis 3, dadurch gekennzeichnet, daß die Verankerungseinrichtung ein Knoten, eine Schraube, ein Gewinde usw. ist.

5. Vorrichtung nach Anspruch 1, dadurch gekennzeichnet, daß die Verankerungseinrichtung und der Stab aus einem Metall und/oder einer Metallegierung hergestellt sind, das bzw. die für den Körper verträglich ist.

6. Vorrichtung nach Anspruch 1, dadurch gekennzeichnet, daß die Verankerungseinrichtung und der Stab aus Edelstahl hergestellt sind.

7. Vorrichtung nach den Ansprüchen 1 bis 4, dadurch gekennzeichnet, daß die Verankerungseinrichtung und der Stab aus Kunststoff hergestellt sind, der für den Körper verträglich ist.

8. Vorrichtung nach den Ansprüchen 1 bis 4 und 7, dadurch gekennzeichnet, daß die Verankerungseinrichtung und der Stab aus Polytetrafluorethylen hergestellt ist.

9. Intrauterine Vorrichtung gemäß den Ansprüchen 1 und 3 bis 8, dadurch gekennzeichnet, daß sie eine enpfängnisverhütende Vorrichtung ist.

10. Vorrichtung nach Anspruch 9, dadurch gekennzeichnet, daß der längliche Stab der Vorrichtung mit einer Einrichtung zur Verhinderung der Befruchtung in Form einer Kupferdrahtspirale oder einer Kupferschicht oder Kupferringen oder -hülsen versehen ist.

11. Vorrichtung nach den Ansprüchen 1 und 3 bis 8, dadurch gekennzeichnet, daß sie eine Vorrichtung zur Strahlungsbehandlung des Uterus ist.

12. Vorrichtung nach den Ansprüchen 1 und 3 bis 8, dadurch gekennzeichnet, daß sie eine Vorrichtung ist, die mit Arzneimitteln gegen Infektionen im Uterus beladen ist.

13. Intravaginale Vorrichtung nach den Ansprüchen 1 und 3 bis 8, dadurch gekennzeichnet, daß die Vorrichtung eine empfängnisverhütende Vorrichtung ist.

14. Vorrichtung nach Anspruch 13, dadurch gekennzeichnet, daß der längliche Stab mit Kupfer oder Hormonen oder mit anderen Arzneimitteln beladen ist.

15. Vorrichtung nach Anspruch 13, dadurch gekennzeichnet, daß der Stab mit einer Schwammscheibe versehen ist, die mit Spermizid zusammen mit oder ohne einem antibiotischen, einem antimykotischen Arzneimittel usw. beladen ist.

16. Vorrichtung nach den Ansprüchen 1 und 3 bis 6, dadurch gekennzeichnet, daß sie eine Vorrichtung zur Behandlung der Inkontinenz ist, und daß sie zu diesem Zweck mit einem Ring mit einem Durchmesser derart versehen ist, daß die Harnröhre aufwärts geschoben wird.

## Revendications

1. Dispositif intra-utérin et/ou intravaginal comprenant une tige allongée qui est munie de moyens d'ancrage, caractérisé en ce que lesdits moyens d'ancrage font partie intégrante de la tige et sont construits afin de percer le col de l'utérus.

2. Dispositif intra-utérin selon la revendication 1, caractérisé en ce que la tige allongée est construite pour être ancrée dans le col de l'utérus approximativement en son milieu de telle sorte que la moitié environ de la tige dépasse dans l'utérus ou bien dans le canal utérin, tandis que l'autre moitié se trouve dans le vagin.

3. Dispositif intra-utérin ou intravaginal selon la revendication 1, caractérisé en ce que l'une des extrémités de la tige est construite pour être ancrée dans le col de l'utérus.

4. Dispositif selon les revendications 1 à 3, caractérisé en ce que les moyens d'ancrage sont un noeud, une vis, un fil, etc.

5. Dispositif selon la revendication 1, caractérisé en ce que le dispositif d'ancrage et la tige sont faits d'un métal et/ou d'un alliage métallique qui est acceptable pour l'organisme.

6. Dispositif selon la revendication 1, caractérisé en ce que les moyens d'ancrage et la tige sont en acier inoxydable.

7. Dispositif selon les revendications 1 à 4, caractérisé en ce que les moyens d'ancrage et la tige sont faits d'un plastique qui est acceptable pour l'organisme.

8. Dispositif selon les revendications 1 à 4 et 7, caractérisé en ce que les moyens d'ancrage et la tige sont en PTFE.

9. Dispositif intra-utérin selon les revendications 1 et 3 à 8, caractérisé en ce qu'il s'agit d'un contraceptif.

10. Dispositif selon la revendication 9, caractérisé en ce que la tige allongée du dispositif est munie de moyens destinés à empêcher la fertilisation sous la forme d'une spirale de fil de cuivre, ou d'une couche de cuivre, ou d'anneaux ou de gaines en cuivre.

11. Dispositif selon les revendications 1 et 3 à 8, caractérisé en ce qu'il s'agit d'un dispositif de traitement de l'utérus par rayonnement.

12. Dispositif selon les revendications 1 et 3 à 8, caractérisé en ce qu'il s'agit d'un dispositif chargé de médicaments destinés à combattre les infections de l'utérus.

13. Dispositif intravaginal selon les revendications 1 et 3 à 8, caractérisé en ce que le dispositif est un contraceptif.

14. Dispositif selon la revendication 13, caractérisé en ce que la tige allongée est chargée avec du cuivre ou des hormones ou d'autres médicaments.

15. Dispositif selon la revendication 13, caractérisé en ce que la tige est munie d'un disque en éponge chargé avec un spermicide avec ou sans antibiotique, un médicament antimycotique, etc.

16. Dispositif selon les revendications 1 et 3 à 8, caractérisé en ce qu'il s'agit d'un dispositif destiné au traitement de l'incontinence et qu'il est muni dans ce but d'un anneau d'un diamètre tel que l'urètre est poussé vers le haut.
